Europäisches Patentamt

European Patent Office

Office européen des brevets

(19)

(11) Publication number: **0 096 403**
**B1**

# EUROPEAN PATENT SPECIFICATION

(12)

(45) Date of publication of patent specification: **05.03.86**

(21) Application number: **83105542.1**

(22) Date of filing: **06.06.83**

(51) Int. Cl.⁴: **C 07 C 19/05, C 07 C 17/10**

(54) **Selective photochlorination of 1,1-dichloroethane with iodine catalyst.**

(30) Priority: **07.06.82 US 385638**

(43) Date of publication of application:
**21.12.83 Bulletin 83/51**

(45) Publication of the grant of the patent:
**05.03.86 Bulletin 86/10**

(84) Designated Contracting States:
**BE DE FR IT NL SE**

(56) References cited:
**GB-A-2 049 668**

(73) Proprietor: **THE DOW CHEMICAL COMPANY
2030 Dow Center Abbott Road P.O. Box 1967
Midland, MI 48640 (US)**

(72) Inventor: **Stevens, James Carl
3 Dan Court
Midland Michigan, 48640 (US)**
Inventor: **Perettie, Donald Joseph
3012 Travis
Midland Michigan, 48640 (US)**

(74) Representative: **Casalonga, Axel et al
BUREAU D.A. CASALONGA OFFICE JOSSE &
PETIT Baaderstrasse 12-14
D-8000 München 5 (DE)**

Courier Press, Leamington Spa, England.

## Description

This invention relates to a photocatalytic process for producing 1,1,1 - trichloroethane by reacting 1,1 - dichloroethane with chlorine.

1,1,1,-Trichloroethane is commonly produced by the hydrochlorination of vinylidene chloride or the chlorination of 1,1 - dichloroethane. The latter has been performed by either thermal chlorination or photochlorination. The photochlorination is presently performed by irradiating mixtures of 1,1 - dichloroethane and chlorine with broad spectrum lamps.

The major problem in such chlorination reactions is the production of by-products, mainly 1,1,2 - trichloroethane. Other by-products produced include 1,1,2,2 - tetrachloroethane, 1,1,1,2 - tetrachloroethane and 1,1,1,2,2 - pentachloroethane. In the conventional chlorination reactions, in excess of 30 percent of the compounds prepared forms these by-products. Mintz, USP 3,580,831, teaches that catalytic amounts of iodine improve the selectivity of photochlorination of mono- and 1,1 - dichloroethane with actinic light.

In Okado et al., Japanese Patent 55—079,329 (1980), the use of iodine and iodine compounds to improve the selectivity of chlorination of 1,1 - dichloroethane for 1,1,1 - trichloroethane is taught. It is further taught that the use of iodine or iodine compounds in liquid phase photochlorination results in especially good selectivity. The photochlorination is accomplished with a broad spectrum of light which includes radiation of a wide range of wavelengths.

Okado et al., German Patent 3,011,689, teaches the use of iodine and iodine compounds as catalysts in vapor phase photochlorination of 1,1 - dichloroethane and that these catalysts improve selectivity. Again, this photochlorination uses a broad spectrum of light of a wide range of wavelengths.

GB—A—2.049.668 ("Asahi Glass Company") teaches the use of iodine and iodine compounds as catalyst in a vapor phase coexisting with a liquid phase photochlorination of 1,1 - dichloroethane for producing 1,1,1 - trichloroethane. The photochlorination is accomplished with a broad range of wavelength of 2500 to 5000 Å from a fluorescent lamp or from a 100 W mercury discharge lamp (which produces significant amount of light from 1367 nm (infrared) to 222.4 nm (for ultraviolet).

## Summary of the invention

The invention is a selective process for preparing 1,1,1 - trichloroethane comprising irradiating a vaporous mixture comprising 1,1 - dichloroethane, chlorine and a photocatalytic amount of iodine or a compound which liberates iodine in the presence of chlorine with radiation from a light source capable of providing radiation at a wavelength comprised between 350 and 550 nm, preferably between 400 and 550 nm and more preferably between 450 and 550 nm at which a binary intermediate formed from iodine and chlorine absorbs much more than chlorine does, said radiation being capable of inducing the selective formation of 1,1,1 - trichloroethane.

This process results in a reaction with surprisingly higher selectivity for 1,1,1 - trichloroethane.

It has been discovered that the use of iodine or an iodine-liberating compound as a photocatalyst significantly increases the selectivity of the photochlorination reaction of 1,1 - dichloroethane and chlorine for 1,1,1 - trichloroethane. Iodine readily reacts with chlorine to form a binary compound, called a binary intermediate herein. It is believed that the binary intermediate is ICl.

When iodine or an iodine-liberating compound is added to the 1,1 - dichloroethane which is contacted with chlorine and then exposed to radiation of a wavelength comprised between 350 and 550 nm at which the binary intermediate formed absorbs, the selectivity of the reaction for 1,1,1 - trichloroethane is significantly increased.

Suitable photocatalysts include iodine or a compound which liberates iodine in the presence of chlorine. Any compound which liberates iodine in the presence of chlorine under the reaction conditions can be used in this invention. Examples of iodine-liberating compounds include hydrogen iodide; alkali metal iodides such as lithium iodide, sodium iodide, potassium iodide, rubidium iodide and cesium iodide; alkaline earth metal iodides such as beryllium iodide, magnesium iodide, barium iodide, calcium iodide or strontium iodide; organic iodides such as methyl iodide, ethyl iodide, propyl iodide, butyl iodide, methylene iodide, iodoform, ethylidene iodide, benzene iodide, benzene di-iodide, toluene iodide or phenol iodide.

The term organic iodide includes aliphatic iodides, cycloaliphatic iodides and aromatic iodides. Aliphatic iodides include alkyl iodides, alkenyl iodides and alkynyl iodides. Aromatic iodides include all iodine compounds which contain an aryl group. The term aryl refers herein to biaryl, biphenylyl, phenyl, naphthyl, phenanthranyl, anthranyl and two aryl groups bridged by an alkylene group. Alkyl includes straight- and branched-chain methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl groups.

Preferred photocatalysts include iodine; hydrogen iodide and organic iodides. More preferred photocatalysts include iodine, hydrogen iodide and alkyl iodides. Even more preferred photocatalysts include iodine and hydrogen iodide, with iodine most preferred.

The above reaction is initiated by exposing the reactants and catalyst to radiation from a suitable source of light. Suitable sources of light include those which are capable of providing radiation at wavelengths where the binary intermediate absorbs much more than chlorine does. It is preferable to use those light sources capable of providing monochromatic light, that is, radiation in a narrow wavelength band. Suitable light sources include lasers and mercury arc lamps fitted with appropriate filters. It is believed that photo-excitation of chlorine increases the formation of by-products, whereas photo-excitation of the binary intermediate without photo-excitation of the chlorine results in formation of less by-products. The processes described in the Background of the Invention use light which produces radiation of a wide range of wavelengths, wherein such range includes those wavelengths at which chlorine absorbs, thus increasing the formation of by-products.

Suitable wavelengths of radiation capable of inducing the selective formation of 1,1,1 - trichloroethane of this reaction are between about 350 and 550 nanometers. It is preferable to use radiation at wavelengths between about 400 and 550 as chlorine has a very low absorption in the lower end of this range. It is most preferable to use radiation at wavelengths between about 450 and 550 nanometers as chlorine does not absorb appreciably in this range. Very low absorption as used herein means that there is some noticeable absorption by chlorine at that wavelength but it is so low that there is very little photo-excitation of chlorine and resultant by-product formation. Does not absorb appreciably as used herein means that there is no noticeable absorption by chlorine although there may actually be some negligible amount of absorption occurring. This reaction can be optimized by using light which provides radiation at a wavelength at which the binary intermediate has its highest absorption and chlorine does not absorb. It has been discovered that when iodine or an iodine-liberating compound is used as a photocatalyst these optimum wavelengths are in the range between about 400 and 550 nanometers, preferably between about 450 and 550 nanometers as the iodine chlorine binary intermediate has a higher absorption in this range.

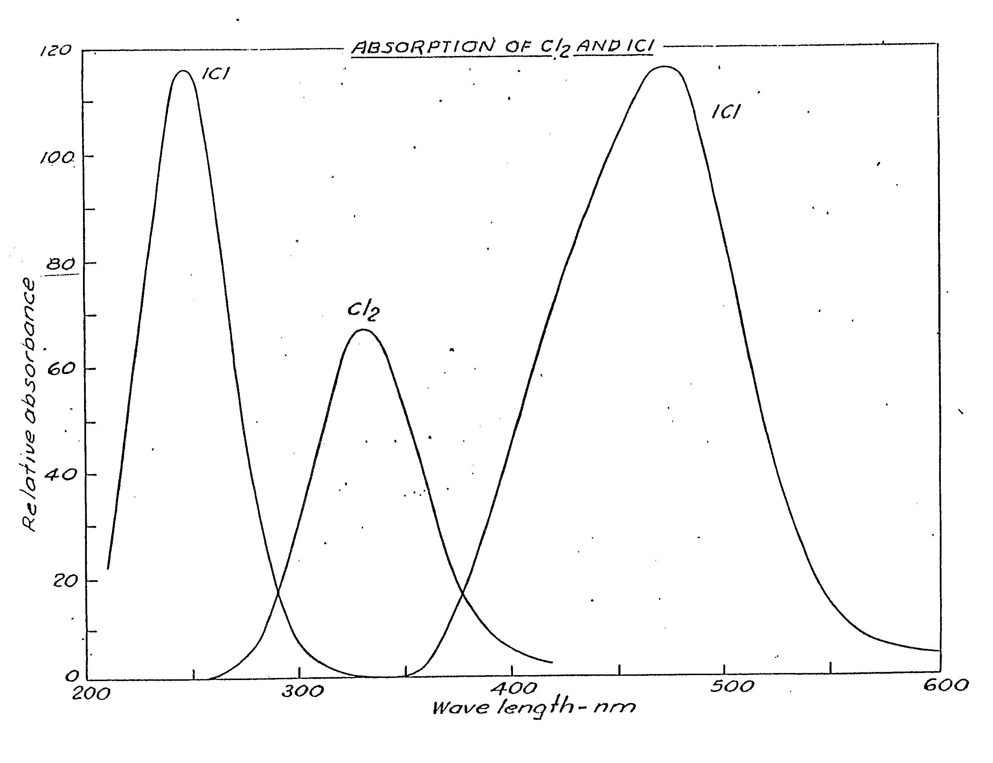

The Figure illustrates those wavelengths at which chlorine absorbs and the iodine chlorine binary intermediate absorbs. It is shown that the iodine/chlorine intermediate absorbs between 350 and 550 nanometers, and that in the lower end of the range between 400 and 550 nanometers, chlorine has a very low absorption and does not absorb appreciably in the higher end. It is further shown that between 450 and 550 nanometers the iodine chlorine intermediate has its highest absorption and chlorine does not absorb appreciably.

It has been discovered that the selectivity of the chlorination of 1,1 - dichloroethane for 1,1,1 - trichloroethane is surprisingly improved wherein iodine or an iodine-liberating compound is used as a photocatalyst and the reaction mixture is exposed to monochromatic light within the above-described ranges. This is an improvement in that the problems of the processes of the prior art are alleviated.

The photocatalyst is used in amounts in which the selectivity of the chlorination of 1,1 - dichloroethane to 1,1,1 - trichloroethane is increased.

Preferably the photocatalyst is employed in amounts which provide between about 50 and 5000 parts of iodine per million parts of 1,1 - dichloroethane by weight, more preferably between about 100 and 2500 parts per million and most preferably between about 500 and 1500 parts per million.

Between about 0.05 and 2.0 moles of chlorine per mole of dichloroethane can be used. It is preferable to use between 0.1 and 0.3 mole of chlorine per mole of 1,1 - dichloroethane, as the selectivity of this reaction improves as the ratio of chlorine to 1,1 - dichloroethane decreases. Further, if the ratio of chlorine to dichloroethane is too high, overchlorination takes place.

The process described herein is a vapor phase reaction which is run at a temperature at which the reactants are in the vapor state. Preferable temperatures are between about 50°C and 400°C, more preferable temperatures are between about 80°C to 150°C. The reactants are usually preheated to insure they are in the vapor phase before exposing them to the desired wavelengths of light.

The reaction may be run at subatmospheric, atmospheric and superatmospheric pressures provided the reactants are in the vapor state.

The following examples are included for merely descriptive purposes and are not intended to limit the scope of the invention.

The mix of products prepared in the following examples is given by a ratio $(\alpha/\beta)$ wherein $\alpha$ is the amount of 1,1,1 - trichloroethane produced and $\beta$ is the amount of 1,1,2 - trichloroethane.

Example 1

1,1-Dichloroethane containing 1097 ppm $I_2$ was pumped into a preheater at about 0.43 g/min. Chlorine gas was fed into the preheater at about 0.8 mmol/min. The reactants were pumped into a reactor after being preheated to 120°C. The reactor was irradiated with an argon ion laser set to deliver 2.0 watts at 488 nanometers. Gas chromatographic analysis of the product collected in the trap showed an $\alpha/\beta$ ratio of 8.6.

Example 2

The photochemical reactor was the same as in Example 1. 1,1 - Dichloroethane (0.43 g/min) (containing 1097 ppm $I_2$) and $Cl_2$ (0.8 mmol/min) were fed into the preheater and preheated to 120°C. Thereafter the preheated mixture was passed into the reactor. The reactor was irradiated with a krypton ion laser set to deliver 2.0 watts at 350 nanometers. Gas chromatographic analysis of the product collected in the trap indicated an $\alpha/\beta$ ratio of 5.9.

Example 3

The photochemical reactor was the same as in Example 1. 1,1 - Dichloroethane (0.43 g/min) (containing 1097 ppm $I_2$) and $Cl_2$(0.8 mmol/min) were fed into the preheater and preheated to 120°C. Thereafter the mixture was irradiated in the reactor using a krypton ion laser set to deliver 2.0 watts at 413 nanometers. Gas chromatographic analysis of the product indicated an α/β ratio of 8.2.

## Claims

1. A selective process for preparing 1,1,1 - trichloroethane comprising photoirradiating a vaporous mixture comprising 1,1 - dichloroethane, chlorine and a photocatalytic amount of iodine or a compound which liberates iodine in the presence of chlorine, the improvement of which comprises the use of radiation between 350 and 550 nanometers.

2. The process of Claim 1 wherein the photocatalyst is iodine, hydrogen iodide, an alkali metal iodide, an alkaline earth metal iodide or an organic iodide.

3. The process of Claim 1 wherein the photocatalyst is iodine, hydrogen iodide or an organic iodide.

4. The process of Claim 1 wherein the photocatalyst is iodine, hydrogen iodide or an alkyl iodide.

5. The process of Claim 1 wherein the photocatalyst is iodine or hydrogen iodide.

6. The process of Claim 1 wherein the photocatalyst is iodine.

7. The process of Claim 1 wherein the light source provides radiation at a wavelength between 400 and 550 nanometers.

8. The process of Claim 1 wherein the light source provides radiation at a wavelength of between 450 and 550 nanometers.

9. The process of Claim 1 wherein an effective amount of photocatalyst is that which provides between about 50 and 5000 parts of iodine per million parts of 1,1 - dichloroethane.

10. The process of Claim 1 wherein an effective amount of photocatalyst is that which provides between about 100 and 2500 parts of iodine per million parts of 1,1 - dichloroethane.

11. The process of Claim 1 wherein the light source is monochromatic.

12. The process of Claim 1 wherein the amount of chlorine contacted with the 1,1 - dichloroethane is between about 0.05 and 2.0 moles of chlorine per mole of 1,1 - dichloroethane.

13. The process of Claim 6 wherein the amount of chlorine contacted with the 1,1 - dichloroethane is between about 0.1 and 0.3 mole of chlorine per mole of 1,1 - dichloroethane.

## Revendications

1. Procédé sélectif pour la préparation du 1,1,1 - trichloro - éthane comprenant la photo - irradiation d'un mélange gazeux comprenant de 1,1 - dichloroéthane, du chlore et une quantité photocatalytique d'iode ou d'un composé qui libère de l'iode en présence de chlore, dont le perfectionnement comprend l'utilisation de radiation entre 350 et 500 nm.

2. Procédé selon la revendication 1, dans lequel le photocatalyseur est l'iode, l'iodure d'hydrogène, un iodure de métal alcalin, un iodure de métal alcalino-terreux ou un iodure organique.

3. Procédé selon la revendication 1, dans lequel le photocatalyseur est l'iode, l'acide iodhydrique ou un iodure organique.

4. Procédé selon la revendication 1, dans lequel le photocatalyseur est l'iode, l'acide iodhydrique ou un iodure d'alkyle.

5. Procédé selon la revendication 1, dans lequel le photocatalyseur est l'iode ou l'acide iodhydrique.

6. Procédé selon la revendication 1, dans lequel le photocatalyseur est l'iode.

7. Procédé selon la revendication 1, dans lequel la source de lumière fournit une radiation à une longueur d'onde entre 400 et 550 nm.

8. Procédé selon la revendication 1, dans lequel la source de lumière fournit une radiation à une longueur d'onde entre 450 et 550 nm.

9. Procédé selon la revendication 1, dans lequel une quantité efficace de photocatalyseur fournit entre environ 50 et 5000 parties d'iode par million de parties de 1,1 - dichloro - éthane.

10. Procédé selon la revendication 1, dans lequel une quantité efficace de photocatalyseur fournit entre environ 100 et 2500 parties d'iode par million de parties de 1,1 - dichloro - éthane.

11. Procédé selon la revendication 1, dans lequel la source de lumière est monochromatique.

12. Procédé selon la revendication 1, dans lequel la quantité de chlore mise en contact avec le 1,1 - dichloroéthane est comprise entre environ 0,05 et 2,0 moles de chlore par mole de 1,1 - dichloro-éthane.

13. Procédé selon la revendication 6, dans lequel la quantité de chlore mise en contact avec le 1,1 - dichloro-éthane est comprise entre environ 0,1 et 0,3 mole de chlore par mole de 1,1 - dichloro - éthane.

## Patentansprüche

1. Ein selektiver Prozeß zur Darstellung von 1,1,1 - Trichloroäthan umfassend die Photobestrahlung eines gasformigen Gemisches bestehend aus 1,1 - Dichloroäthan, Chlor und einer photokatalytischen Menge Jod oder einer Verbindung, welche in Gegenwart von Chlor Jod freisetzt, dessen Verbesserung die Verwendung von Strahlen zwischen 350 und 550 Nanometer umfaßt.

2. Der Prozeß vom Anspruch 1, wobei der Photokatalysator Jod, Jodwasserstoff, Jodid eines alkalischen Metalls, Jodid eines erdalkalischen Metalls oder organisches Jod ist.

3. Der Prozeß vom Anspruch 1, wobei der Photokatalysator Jod, Jodwasserstoff oder organisches Jodid ist.

4. Der Prozeß vom Anspruch 1, wobei der Photokatalysator Jod, Jodwasserstoff oder Alkyljodid ist.

5. Der Prozeß vom Anspruch 1, wobei der Photokatalysator Jod oder Jodwasserstoff ist.

6. Der Prozeß vom Anspruch 1, wobei der Photokatalysator Jod ist.

7. Der Prozeß vom Anspruch 1, wobei die Lichtquelle Strahlen mit Wellenlänge zwischen 400 und 550 Nanometer liefert.

8. Der Prozeß vom Anspruch 1, wobei die Lichtquelle Strahlen mit Wellenlänge zwischen 450 und 550 Nanometer liefert.

9. Der Prozeß vom Anspruch 1, wobei eine effektive Menge Photokatalysator diese ist, welche zwischen ungefähr 50 und 5000 Teile Jod für eine Million Teile 1,1 - Dichloroäthan liefert.

10. Der Prozeß vom Anspruch 1, wobei eine effektive Menge Photokatalysator diese ist, welche zwischen ungefähr 100 und 2500 Teile Jod für eine Million Teile 1,1 - Dichloroäthan liefert.

11. Der Prozeß vom Anspruch 1, wobei die Lichtquelle monochromatisch ist.

12. Der Prozeß vom Anspruch 1, wobei die Menge Chlor, welche mit dem 1,1 - Dichloroäthan in Kontakt kommt, zwischen ungefähr 0,05 und 2,0 Mol Chlor für ein Mol 1,1 - Dichloroäthan ist.

13. Der Prozeß vom Anspruch 6, wobei die Menge Chlor, welche mit dem 1,1 - Dichloroäthan in Kontakt kommt, zwischen ungefähr 0,1 und 0,3 Mol Chlor für ein Mol 1,1 - Dichloroäthan ist.

ABSORPTION OF $Cl_2$ AND $ICl$